# EUROPÄISCHE PATENTSCHRIFT

(11) **EP 2 975 112 B1**
(45) Veröffentlichungstag und Bekanntmachung des Hinweises auf die Patenterteilung: **05.09.2018**
(21) Anmeldenummer: 15001964.4
(22) Anmeldetag: 01.07.2015
(51) Int. Cl.: C12M 1/107, C12M 1/00, C12M 1/26

(54) **FERMENTER, ANLAGE UND VERFAHREN ZUR ERZEUGUNG VON BIOGAS**
FERMENTER, PLANT AND METHOD FOR GENERATING BIOGAS
FERMENTEUR, INSTALLATION ET PROCEDE DE PRODUCTION DE BIOGAZ

(30) Priorität: 17.07.2014 DE 102014010641
(43) Veröffentlichungstag der Anmeldung: 20.01.2016
(73) Patentinhaber: Eisenmann SE, 71032 Böblingen (DE)
(72) Erfinder: Schlüter, Thomas, 71093 Weil im Schönbuch (DE)
(74) Vertreter: Ostertag & Partner Patentanwälte mbB

(56) Entgegenhaltungen:
- WO-A2-2010/082741
- FR-A1- 2 519 965
- US-A- 4 342 836
- US-A1- 2013 206 345

## Beschreibung

Die Erfindung betrifft einen Fermenter zur Erzeugung von Biogas durch anaerobe Fermentation mit mindestens einer Fermentierkammer und mit mindestens einer Zuführeinrichtung, wobei die Zuführeinrichtung zum Eintragen von Fermentiergut in die Fermentierkammer ausgebildet ist.

Die Erfindung betrifft auch eine Anlage zur Erzeugung von Biogas mit mindestens einem Fermenter.

Die Erfindung betrifft auch ein Verfahren zur Erzeugung von Biogas durch anaerobe Fermentation eines organischen Fermentierguts in einem Fermenter, wobei das Fermentiergut mittels einer Zuführeinrichtung in eine Fermentierkammer des Fermenters eingetragen wird.

Es ist Stand der Technik, zur Unterstützung des Fermentationsprozesses im Fermenter einer Biogasanlage Wärmeenergie in die Fermentationskammer einzubringen. In diesem Zusammenhang ist es bekannt, Boden-und/oder Wandheizungen einzusetzen. Bei Bodenheizungen, bei denen zum Beispiel Kunststoffheizspiralen in den Fermenterboden verlegt werden, können Sinkschichten zu einer starken Verschlechterung der Wärmeübertragung führen. Bei Wandheizungen ist es bekannt, Kunststoffheizrohre ähnlich wie bei der Bodenheizung in den Beton einzugießen. Eine bessere Wärmeübertragung liefern mit einigem Abstand an der Wand des Fermenters befestigte Edelstahl- oder Kunststoffrohre in zwei oder mehr Heizkreisen. Nachteilig ist bei den beschriebenen Heizungen unter anderem, dass häufig Probleme an den Wärmetauschern auftreten, beispielsweise durch Korrosion, Abrasion, Verkrustungen und/oder Verhängungen. Bei Plattenheizungen sind vergleichbare Probleme bekannt.

Die Druckschrift US4342836 A beschreibt ein System zum anaeroben Aufbereiten organischen Materials zur Herstellung von Methangas. Innerhalb der Biokonvertereinheit wird das organische Material mittels einer Förderschnecke gefördert. Das Dokument beschreibt eine optionale Ausführungsform, bei der ein Fluid, wie Dampf oder Wasser, unter Druck eingebracht werden kann. Aufgabe der vorliegenden Erfindung ist es, einen Fermenter zur Erzeugung von Biogas bereitzustellen, bei dem die Nachteile des Stands der Technik vermieden werden.

Diese Aufgabe wird bei einem Fermenter der eingangs genannten Art dadurch gelöst, dass
a) mindestens ein Dampfinjektor zum Einbringen von Dampf vorgesehen ist;
b) der Dampfinjektor unterhalb der Zuführeinrichtung angeordnet ist und die Zuführeinrichtung und der Dampfinjektor derart angeordnet sind, dass sich das Fermentiergut und der Dampf in räumlicher Nähe der Zuführeinrichtung vermengen.

Dadurch wird ein direkter und vergleichsweise verlustfreier Wärmeübergang bewirkt. Weiterhin kann ein Voraufschluss des Fermentierguts erfolgen.

Mit Vorteil können die Zuführeinrichtung und der mindestens eine Dampfinjektor benachbart zueinander angeordnet sein. Dadurch können insbesondere Wärmeverluste reduziert werden.

Um einen möglichst direkten Wärmeübergang zu begünstigen, ist der Dampfinjektor vorzugsweise auf das die Zuführeinrichtung verlassende Fermentiergut ausgerichtet.

In weiterer Ausgestaltung der Erfindung kann der Dampfinjektor als Dampflanze ausgebildet sein. Derart kann Wärmeenergie gezielt eingebracht werden.

Um Fermentiergut möglichst bedarfsgerecht in den Fermenter einzutragen, kann die Zuführeinrichtung als Förderschnecke für zumindest weitestgehend festes Fermentiergut ausgebildet sein.

Es kann hinsichtlich der Prozessführung von Vorteil sein, den Dampfinjektor unterhalb der Zuführeinrichtung anzuordnen.

Die Prozesseffizienz kann gesteigert werden, wenn der Fermenter als Pfropfenstromfermenter ausgebildet ist.

Die Aufgabe der Erfindung wird auch gelöst durch eine Anlage zur Erzeugung von Biogas mit mindestens einem Fermenter, der gemäß der Erfindung oder gemäß einer Ausführung der Erfindung ausgebildet ist, wobei eine Dampfbereitstellungseinrichtung vorgesehen ist, welche über eine Dampfzuführung mit dem Dampfinjektor gekoppelt ist.

Bevorzugt kann zum Bereitstellen von Dampf ein Gas betriebener Dampferzeuger vorgesehen sein. Derart kann Dampf für den Dampfinjektor mit der prozesstechnisch erforderlichen Temperatur in effizienter Weise bereitgestellt werden.

In vorteilhafter Ausgestaltung kann die Anlage zur Erzeugung von Biogas ein Blockheizkraftwerk mit einem Verbrennungsmotor aufweisen, wobei das Blockheizkraftwerk derart mit der Dampfbereitstellungseinrichtung gekoppelt ist, dass die Abwärme des Verbrennungsmotors zur Dampferzeugung verwendet wird. Auf diese Weise kann die Energieeffizienz der Anlage gesteigert werden.

Die Aufgabe der Erfindung wird auch gelöst durch ein Verfahren der eingangs genannten Art, bei welchem dem Fermentiergut in räumlicher Nähe der Zuführeinrichtung durch Einbringen von Dampf Wärmeenergie zugeführt wird. Dadurch wird ein direkter und vergleichsweise verlustfreier Wärmeübergang bewirkt und es kann ein Voraufschluss des Fermentierguts erfolgen.

Vorzugsweise erfolgt das Zuführen von Wärmeenergie beim Eintreten des Fermentierguts in die Fermentierkammer.

Weitere vorteilhafte Ausgestaltungen ergeben sich aus der nachfolgenden Beschreibung. Dabei werden Ausführungsbeispiele der Erfindung, ohne hierauf beschränkt zu sein, an Hand der Zeichnungen näher erläutert. Es zeigen, jeweils in vereinfachter, schematischer Darstellung:
- Figur 1: einen Teil einer Biogasanlage
- Figur 2: den Aufbau einer Biogasanlage

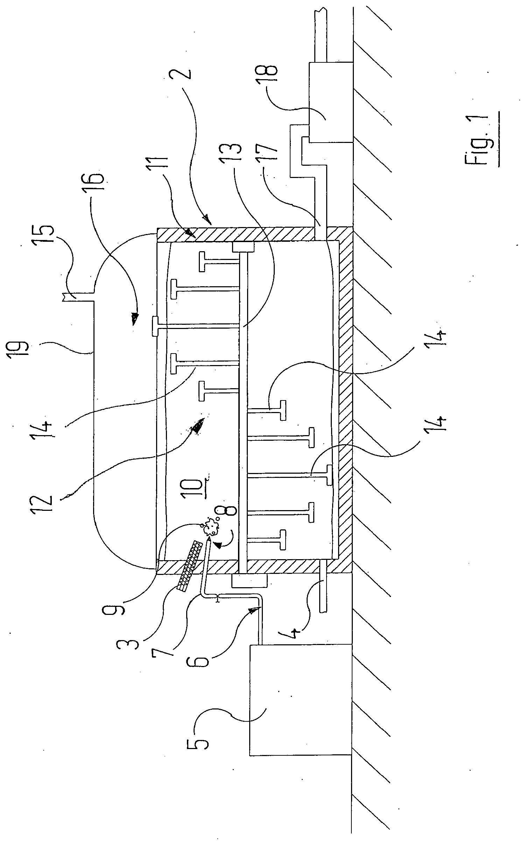

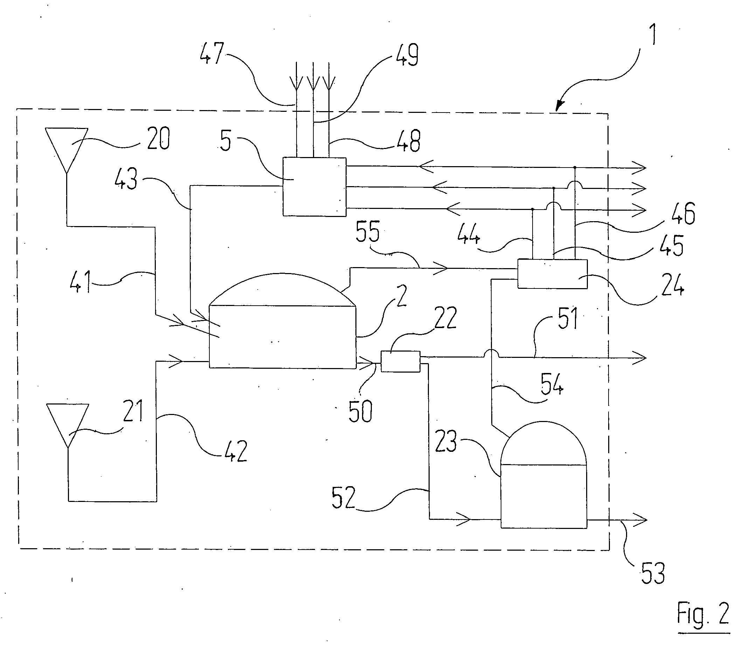

Figur 1 zeigt einen Teil einer Biogasanlage 1 (siehe Figur 2) mit einem Fermenter 2 zur Erzeugung von Biogas durch anaerobe Fermentation organischen Materials.

Der Fermenter 2 weist eine Zuführeinrichtung 3 auf, die zum Eintragen von Fermentiergut in eine Fermentierkammer 10 des Fermenters 2 ausgebildet ist. Im gezeigten Beispiel ist die Zuführeinrichtung 3 wie in der Zeichnung angedeutet als Förderschnecke ausgebildet. Diese Förderschnecke dient als Eintragsschnecke für festes Fermentiergut 41 (siehe Figur 2). Festes Fermentiergut 41 bzw. Festsubstrat kann beispielsweise aus der Bioabfall-Trennung zugeführt werden. Festes Fermentiergut 41 kann beispielsweise auch Biomasse landwirtschaftlicher Herkunft sein, wie z.B. Mais, Grasschnitt, Futterrüben, andere Pflanzen und/oder Silage.

Im gezeigten Beispiel weist der Fermenter 2 eine Zuführleitung 4 für flüssiges Fermentiergut 42 (siehe Figur 2) auf. Flüssiges Fermentiergut 42 bzw. Flüssigsubstrat wird in Biogasanlagen als Grundsubstrat eingesetzt, wobei beispielsweise Flüssigmist als flüssiges Fermentiergut 42 verwendet werden kann.

Im Fermenter 2 wird energetisch verwertbares Biogas gewonnen, wobei als Fermenter 2 Behälter unterschiedlichen geometrischen Aufbaus zum Einsatz kommen können, welche beispielsweise liegend oder stehend angeordnet sein können. Als Materialen zur Fertigung eines Fermenterbehälters können beispielsweise Beton und/oder Stahl, insbesondere Edelstahl, verwendet werden. Stehende Fermenterbehälter können beispielsweise mit einem runden Querschnitt ausgeführt sein. Liegende Fermenterbehälter können beispielsweise einen rechteckigen oder quadratischen Querschnitt aufweisen. Liegende Fermenterbehälter können beispielsweise auch im Wesentlichen zylindrisch ausgebildet sein.

Der in Figur 1 schematisch dargestellte Fermenter 2 kann z.B. als sogenannter Pfropfenstromfermenter ausgebildet sein, wobei der Inhalt des Fermenters 2 auf Grund des Verdrängungseffekts des eingebrachten Materials - ähnlich einem Pfropfen -in horizontaler Richtung durch die Fermentierkammer 10 des Fermenters 2 transportiert wird. Daraus resultieren eine höhere Belastbarkeit, geringe Rezirkulation sowie positive Effekte auf den Abbaugrad und die Hygienisierbarkeit des Materials.

Im gezeigten Beispiel ist auf der Seite des Gehäuses 11 des Fermenters 2, welche Gehäuseseite der Zuführeinrichtung 3 und der Zuführleitung 4 gegenüberliegt, eine Gärresteaustragseinrichtung 17 vorgesehen. Im Beispiel ist diese Gärresteaustragseinrichtung 17 mit einer Pumpe 18 gekoppelt.

In der Fermentierkammer 10 ist ein Rührwerk 12 vorgesehen. Das Rührwerk 12 weist eine Rührwelle 13 auf, an der nur teilweise mit Bezugszeichen versehene Rührwerkzeuge 14 angeordnet sind. Mittels des Rührwerks 12 erfolgt eine Durchmischung des Fermenterinhalts, wobei die Bildung von Schwimm- und Sinkschichten verringert wird und die mikrobiologische Aktivität im Fermenter positiv beeinflusst wird. Im gezeigten Beispiel ist das Rührwerk 12 als Haspelrührwerk ausgebildet, wobei die Rührwerkzeuge 14 als Rührarme entlang der Rührwelle 13 angeordnet sind. Im gezeigten Beispiel ragen die Rührwerkzeuge 14 von der Rührwelle 13 radial nach außen. Es kann vorteilhaft sein, wenn die Rührwerkzeuge 14 zumindest in einem Teilbereich des Fermenters 2 spiralförmig angeordnet sind und - wie in der Zeichnung angedeutet - an ihren Enden mit kurzen Paddeln ausgestattet sind.

Der Fermenter 2 kann als Hauptfermenter ausgebildet sein, dem ein Nachfermenter, der auch als Nachgärbehälter bezeichnet werden kann, nachgeordnet sein kann. Bei Vorsehen eines Nachfermenters kann dieser unter Verwendung der Gärresteaustragseinrichtung 17 mit dem Hauptfermenter gekoppelt sein. In den Zeichnungen nicht näher dargestellt ist die Möglichkeit, einen kontinuierlich betriebenen Fermenter 2 mit einer Hauptfermentierkammer und mit einer Nachfermentierkammer auszustatten. Diese Möglichkeit wird z.B. in der DE 20 2006 013772 U1 beschrieben.

Das Biogas, welches im Fermenter 2 entsteht, sammelt sich in einem Gasspeicher 16 unterhalb einer Abdeckung 19. Wie im Beispiel schematisch gezeigt, wird der Gasspeicher 16 nach oben durch die Abdeckung 19 und nach unten durch den Spiegel des in der Fermentierkammer 10 befindlichen Materials begrenzt. Die Abdeckung 19 kann beispielsweise als Folienhaube aus flexiblem, gasdichten Material ausgebildet sind. Eine andere Ausführungsform der Abdeckung 19 ist beispielsweise ein sogenanntes Tragluftdach oder eine separate Betondecke. Bei einem Tragluftdach ist in der Regel eine Außenmembrane vorgesehen unterhalb derer eine Gasspeichermembrane angeordnet ist, durch welche der Gasspeicher nach oben begrenzt wird. Alternativ oder zusätzlich zur gezeigten Anordnung des Gasspeichers 16 ist es auch möglich einen gesonderten Speicher zur Gaslagerung vorzusehen. Ein gesonderter Speicher kann z.B. ein externer Foliengasspeicher sein.

Mittels einer in der Zeichnung lediglich angedeuteten Gasentnahmeeinrichtung 15 kann dem Gasspeicher 16 Biogas zur Nutzung entnommen werden. Eine der Nutzungsarten ist es beispielsweise, das im Fermenter gewonnene Biogas 55 einem Blockheizkraftwerk 24 zuzuführen (siehe Figur 2). Im Blockheizkraftwerk 24 kann das Biogas 55 verstromt werden, beispielsweise um einen monetären Ertrag durch Einspeisung zu erwirtschaften. Der Eigenstrombedarf der Biogasanlage 1 (siehe Figur 2) kann aus dem Stromnetz und/oder zumindest teilweise durch das Blockheizkraftwerk 24 gedeckt werden.

Im gezeigten Beispiel ist der Fermenter 2 mit einem Dampfinjektor 7 ausgestattet, der im selben Bereich des Gehäuses 11 angeordnet ist, in dem auch die Zuführeinrichtung 3 angeordnet ist. Die Zuführeinrichtung 3 und der Dampfinjektor 7 sind benachbart zueinander angeordnet. Der Dampfinjektor 7 ist auf das von der Zuführeinrichtung 3 in die Fermentierkammer 10 eingetragene feste Fermentiergut 41 gerichtet. Wie in der Zeichnung angedeutet, kann der Dampfinjektor 7 beispielsweise als Dampflanze ausgebildet sein und eine düsenförmige Austrittsöffnung 8 für Dampf aufweisen. Es können auch mehrere auf das Fermentiergut gerichtete Dampflanzen vorgesehen sein. Figur 1 zeigt ein Beispiel für einen als Dampflanze ausgebildeten Dampfinjektor 7, der unterhalb der Zuführeinrichtung 3 für festes Fermentiergut 41 angeordnet ist.

Eine Dampfbereitstellungseinrichtung 5 stellt Dampf 43 (siehe Figur 2), d.h. Wasserdampf, für den Dampfinjektor 7 bereit. Der Wasserdampf wird dem Dampfinjektor 7 mittels einer Dampfzuführung 6 zugeführt. Der Wasserdampf wird als Nassdampf, ggf. auch als Sattdampf bzw. gesättigter Dampf, bereitgestellt. Der Dampf, d.h. der Wasserdampf, kann beispielsweise eine Temperatur in einem Bereich von ungefähr 145°C bis ungefähr 180°C aufweisen, z.B. ca. 165°C. Die Dampfbereitstellungseinrichtung 5 kann ein Dampferzeuger sein, der elektrisch und/oder mit Gas betrieben werden kann. Die Dampfbereitstellungseinrichtung 5 kann z.B. als Schnelldampferzeuger ausgebildet sein.

Mit Hilfe des Dampfinjektors 7 wird Wärmeenergie in direkter räumlicher Nähe zum Substrateintrag in den Fermenter eingebracht. Fermentiergut 41 und Dampf 43 vermengen sich im Bereich des Dampfeintritts 9 bzw. in dessen unmittelbarer Umgebung, wobei sich der Bereich des Dampfeintritts 9 in räumlicher Nähe der Zuführeinrichtung 3 befindet. Dadurch, dass sich das Fermentiergut 41 und der Dampf 43 in räumlicher Nähe der Zuführeinrichtung vermengen wird ein direkter und vergleichsweise verlustfreier Wärmeübergang auf das Fermentiergut bewirkt.

Der Dampfinjektor 7 ist auf das die Zuführeinrichtung 3 verlassende feste Fermentiergut 41 gerichtet. Dabei kann durch die lokal begrenzt hohen Temperaturen ein Voraufschluss des Fermentierguts 41 erfolgen. Im gezeigten Beispiel wird dem Fermentiergut 41 beim Eintreten bzw. unmittelbar oder zumindest nahezu unmittelbar nach dem Eintreten in den Fermenter 2 Wärmeenergie zugeführt. Es können weitere, nicht näher dargestellte Dampfinjektoren vorgesehen sein, die auf das Fermentiergut 41 gerichtet sind.

Die Dampfzuführung kann kontinuierlich oder getaktet zum Substrateintrag erfolgen. Bei einer taktweisen Dampfzuführung können das Substrat und der Dampf sowohl gemeinsam als auch alternierend und nicht gemeinsam zugeführt werden.

Figur 2 zeigt sehr schematisch den Aufbau einer Anlage 1 zur Erzeugung von Biogas. In dieser Anlage 1 entsteht in einem Fermenter 2 Biogas 55 durch anaerobe Fermentation von Fermentiergut 41 bzw. 42, welches als organisches Material vorliegt. Anaerobe Fermentation bedeutet, dass unter Ausschluss von Sauerstoff organische Stoffe durch mikrobiologische Aktivität abgebaut werden.

Im gezeigten Beispiel wird dem Fermenter festes Fermentiergut 41 aus einer ersten Quelle 20 für festes Material und flüssiges Fermentiergut 42 aus einer zweiten Quelle 22 für flüssiges Material zugeführt. Festes Fermentiergut 41, d.h. im Wesentlichen Festsubstrat, kann beispielsweise Silage, Grasschnitt, Materialien aus der Bioabfall-Trennung und/oder Festmist aus der Tierhaltung sein. Als flüssiges Fermentiergut 42, d.h. im Wesentlichen Flüssigsubstrat, können beispielsweise Flüssigmist und/oder Flüssigkeiten aus der Speiseresteaufbereitung, die ggf. einer Pasteurisierung unterzogen werden, verwendet werden.

Der Fermenter 2 ist mit einer Dampfbereitstellungseinrichtung 5 gekoppelt, wobei der Fermenter 2 mindestens einen Dampfinjektor 7 (siehe Figur 1) aufweist, welchem von der Dampfbereitstellungseinrichtung 5 bereitgestellter Dampf 43 zugeführt wird.

Das Biogas 55 aus dem Fermenter 2 wird im gezeigten Beispiel einem Blockheizkraftwerk 24 zugeführt.

Gärreste 50 aus dem Fermenter 2 können als Düngemittel 51 und/oder Kompost verwendet werden. In alternativer oder zusätzlicher Verwendung können Gärreste 50 aus dem Fermenter 2 einem Gärrestelager 23 zugeführt werden. Im gezeigten Beispiel ist dem Fermenter 2 eine Separatorvorrichtung 22 für die Gärreste 50 nachgeordnet. Mittels der Separatorvorrichtung 22 können Düngemittel 51 und/oder Kompost von übrigen Gärresten 52, die dem Gärrestelager 23 zugeführt werden, getrennt werden. Dem Gärrestelager 23 kann Gülle 53 entnommen werden, welche beispielsweise zum Düngen in der Landwirtschaft eingesetzt werden kann. Auch im Gärrestelager 23 kann nutzbares Biogas 54 entstehen, welches bei entsprechender Ausgestaltung des Gärrestelagers dem Blockheizkraftwerk 24 zugeführt werden kann.

Im Blockheizkraftwerk 24, das vorzugsweise modular aufgebaut ist, werden aus dem Biogas 55, 54 elektrische Energie 46 und Wärme 45 gewonnen. Elektrische Energie 46 und Wärme 45 können im Blockheizkraftwerk 24 beispielsweise unter Verwendung eines als Gasmotor ausgebildeten Verbrennungsmotors erzeugt werden. Zur Gewinnung von elektrischer Energie 46 kann der mindestens eine Verbrennungsmotor des Blockheizkraftwerks 24 mit einem oder mehreren Generatoren gekoppelt sein.

Die in dem Blockheizkraftwerk 24 gewonnene elektrische Energie 46 kann außerhalb der Anlage 1 genutzt und hierzu in ein Energienetz eingespeist werden. Alternativ oder ergänzend kann die gewonnene elektrische Energie 46 wieder in die Anlage 1 zurückgeführt werden und dort zur Energieversorgung von elektrischen Verbrauchern dienen, welche zum Betrieb der Anlage 1 vorhanden sind.

Beispielsweise kann zumindest ein Teil der elektrischen Energie 46 der Dampfbereitstellungseinrichtung 5 zugeführt werden. Alternativ oder zusätzlich zu der vom Blockheizkraftwerk 24 bereitgestellten elektrischen Energie 46, kann der Dampfbereitstellungseinrichtung 5 elektrische Energie 49 aus anderer Quelle zugeführt werden.

Die im Blockheizkraftwerk 24 gewonnene Wärme 45 kann außerhalb und/oder innerhalb der Anlage 1 zur Erzeugung von Biogas genutzt werden. So kann die gewonnene Wärme 45 z.B. in ein Nahwärmenetz eingespeist werden bzw. zum Betrieb der Anlage 1 zur Erzeugung von Biogas 54, 55 genutzt werden. Beispielsweise kann zumindest ein Teil der gewonnenen Wärme 45 der Dampfbereitstellungseinrichtung 5 zugeführt werden. Alternativ oder zusätzlich zu der vom Blockheizkraftwerk 24 bereitgestellten Wärme 45, kann der Dampfbereitstellungseinrichtung 5 Wärme 48 aus anderer Quelle zugeführt werden.

Zum Erzeugen von Dampf 43 kann der Dampfbereitstellungseinrichtung 5 Gas 47 zugeführt werden, wobei als Gas 47 Biogas, Erdgas oder auch Flüssiggas verwendet werden kann. Beispielsweise kann ein Schnelldampferzeuger mit Gas 47 betrieben werden. Als Gas 47 kann der Dampfbereitstellungseinrichtung 5 auch Biogas 54, 55 zugeführt werden, welches innerhalb der Anlage 1 zur Erzeugung von Biogas gewonnen wird. Letztere Ausführung ist in der Zeichnung der besseren Übersicht halber nicht näher dargestellt.

Alternativ oder zusätzlich zu der elektrischen Energie 46, 49, der Wärme 45, 48 und/oder dem Gas 47, kann der Dampfbereitstellungseinrichtung 5 auch Dampf zugeführt werden. Außerhalb der Dampfbereitstellungseinrichtung 5 kann Dampf z.B. im Blockheizkraftwerk 24 erzeugt werden. So kann beispielsweise durch die heißen Abgase eines Verbrennungsmotors des Blockheizkraftwerks 24 Dampf erzeugt werden. Die Zuführung von Dampf vom Blockheizkraftwerk 24 zur Dampfbereitstellungseinrichtung 5 ist in der Zeichnung der besseren Übersicht halber nicht näher dargestellt.

Um Dampf 43 für den Dampfinjektor 7 bedarfsgerecht und effizient bereitzustellen, kann unter Zuhilfenahme des Verbrennungsmotors des Blockheizkraftwerks 24 Dampf erzeugt werden und zusätzlich ein Schnelldampferzeuger vorgesehen sein, wobei der Schnelldampferzeuger vorzugsweise Gas betrieben ist.

Ein für die Erfindung wesentlicher Gedanke lässt sich wie folgt zusammenfassen: Die Erfindung betrifft einen Fermenter 2 zur Erzeugung von Biogas durch anaerobe Fermentation organischen Substrats mit mindestens einer Fermentierkammer 10 und mit mindestens einer Zuführeinrichtung 3, welche zum Eintragen von Substrat, insbesondere Festsubstrat, in die Fermentierkammer 10 ausgebildet ist, wobei mindestens ein Dampfinjektor 7 zum Einbringen von Dampf 43 in die Fermentierkammer 10 vorgesehen ist, und wobei die Zuführeinrichtung 3 und der Dampfinjektor 7 derart zueinander angeordnet sind, dass sich das eingetragene Substrat, insbesondere Festsubstrat, und der eingebrachte Dampf 43 in räumlicher Nähe der Zuführeinrichtung 3 vermengen. Ein Vorteil der Erfindung liegt darin, dass dem Substrat, insbesondere Festsubstrat, Wärmeenergie direkt zugeführt wird, wobei Verluste reduziert werden können. Die Erfindung betrifft auch eine entsprechende Anlage 1 zur Erzeugung von Biogas 54, 55 sowie ein Verfahren zur Erzeugung von Biogas 54, 55.

## Patentansprüche

1. Fermenter (2) zur Erzeugung von Biogas durch anaerobe Fermentation mit mindestens einer Fermentierkammer (10) und mit mindestens einer Zuführeinrichtung (3), wobei die Zuführeinrichtung (3) zum Eintragen von Fermentiergut (41) in die Fermentierkammer (10) ausgebildet ist,
**dadurch gekennzeichnet, dass**
a) mindestens ein Dampfinjektor (7) zum Einbringen von Dampf (43) vorgesehen ist;
b) der Dampfinjektor (7) unterhalb der Zuführeinrichtung (3) angeordnet ist und der Dampfinjektor (7) auf das die Zuführeinrichtung (3) verlassende und in die Fermentierkammer (10) eingetragene feste Fermentiergut (41) ausgerichtet ist, so dass sich das Fermentiergut (41) und der Dampf (43) in räumlicher Nähe der Zuführeinrichtung (3) vermengen.

2. Fermenter (2) nach Anspruch 1, **dadurch gekennzeichnet, dass** die Zuführeinrichtung (3) und der mindestens eine Dampfinjektor (7) benachbart zueinander angeordnet sind.

3. Fermenter (2) nach einem der Ansprüche 1 bis 2, **dadurch gekennzeichnet, dass** der Dampfinjektor (7) als Dampflanze ausgebildet ist.

4. Fermenter (2) nach einem der Ansprüche 1 bis 3, **dadurch gekennzeichnet, dass** die Zuführeinrichtung (3) als Förderschnecke für zumindest weitestgehend festes Fermentiergut (41) ausgebildet ist.

5. Fermenter (2) nach einem der Ansprüche 1 bis 4, **dadurch gekennzeichnet, dass** der Fermenter (2) als Pfropfenstromfermenter ausgebildet ist.

6. Anlage zur Erzeugung von Biogas mit mindestens einem Fermenter (2) nach einem der vorangehenden Ansprüche und mit einer Dampfbereitstellungseinrichtung (5), welche über eine Dampfzuführung (6) mit dem Dampfinjektor (7) gekoppelt ist.

7. Anlage zur Erzeugung von Biogas nach Anspruch6, **dadurch gekennzeichnet, dass** zum Bereitstellen von Dampf ein Gas betriebener Dampferzeuger vorgesehen ist.

8. Anlage zur Erzeugung von Biogas nach Anspruch 6 oder 7, **dadurch gekennzeichnet, dass** die Anlage (1) ein Blockheizkraftwerk (24) mit einem Verbrennungsmotor aufweist, wobei das Blockheizkraftwerk (24) derart mit der Dampfbereitstellungseinrichtung (5) gekoppelt ist, dass die Abwärme des Verbrennungsmotors zur Dampferzeugung verwendet wird.

9. Verfahren zur Erzeugung von Biogas (55) durch anaerobe Fermentation eines organischen Fermentierguts (41) in einem Fermenter (2) nach einem der Ansprüche 1-5, wobei das Fermentiergut (41) mittels einer Zuführeinrichtung (3) in eine Fermentierkammer (10) des Fermenters (2) eingetragen wird,
**dadurch gekennzeichnet, dass**
dem Fermentiergut (41) in räumlicher Nähe der Zuführeinrichtung (3) Wärmeenergie durch Einbringen von Dampf (43) zugeführt wird.

10. Verfahren nach Anspruch 9, **dadurch gekennzeichnet, dass** das Zuführen von Wärmeenergie beim Eintreten des Fermentierguts (41) in die Fermentierkammer (10) erfolgt.

## Claims

1. Fermenter (2) for generating biogas by anaerobic fermentation, having at least one fermentation chamber (10) and having at least one feed device (3), wherein the feed device (3) is designed for introducing material to be fermented (41) into the fermentation chamber (10),
**characterized in that**
a) at least one steam injector (7) is provided for introducing steam (43);
b) the steam injector (7) is arranged below the feed device (3), and the steam injector (7) is directed at the solid material to be fermented (41) which is exiting the feed device (3) and being introduced into the fermentation chamber (10), with the result that the material to be fermented (41) and the steam (43) mix in the spatial proximity of the feed device (3).

2. Fermenter (2) according to Claim 1, **characterized in that** the feed device (3) and the at least one steam injector (7) are arranged adjacent to one another.

3. Fermenter (2) according to either of Claims 1 and 2, **characterized in that** the steam injector (7) is designed as a steam lance.

4. Fermenter (2) according to one of Claims 1 to 3, **characterized in that** the feed device (3) is designed as a conveying screw for at least substantially solid material to be fermented (41).

5. Fermenter (2) according to one of Claims 1 to 4, **characterized in that** the fermenter (2) is designed as a plug flow fermenter.

6. Plant for generating biogas, having at least one fermenter (2) according to one of the preceding claims, and having a steam provision device (5) which is coupled to the steam injector (7) via a steam feed (6).

7. Plant for generating biogas according to Claim 6, **characterized in that**, for providing steam, a gas-operated steam generator is provided.

8. Plant for generating biogas according to Claim 6 or 7, **characterized in that** the plant (1) has a cogeneration unit (24) with a combustion engine, wherein the cogeneration unit (24) is coupled to the steam provision device (5) such that the waste heat of the combustion engine is used for generating steam.

9. Method for generating biogas (55) by anaerobic fermentation of an organic material to be fermented (41) in a fermenter (2) according to one of Claims 1-5, wherein the material to be fermented (41) is introduced into a fermentation chamber (10) of the fermenter (2) by means of a feed device (3),
**characterized in that**
heat energy is supplied to the material to be fermented (41) in the spatial proximity of the feed device (3) by the introduction of steam (43).

10. Method according to Claim 9, **characterized in that** the supply of heat energy occurs when the material to be fermented (41) enters the fermentation chamber (10) .

## Revendications

1. Fermenteur (2) pour la production de biogaz par fermentation anaérobie avec au moins une chambre de fermentation (10) et au moins un dispositif d'alimentation (3), dans lequel le dispositif d'alimentation (3) est conçu pour charger des matières à fermenter (41) dans la chambre de fermentation (10),
**caractérisé en ce que**
a) il est prévu au moins un injecteur de vapeur (7) pour l'introduction de vapeur (43);
b) l'injecteur de vapeur (7) est disposé en dessous du dispositif d'alimentation (3) et l'injecteur de vapeur (7) est dirigé vers les matières solides à fermenter (41) quittant le dispositif d'alimentation (3) et chargées dans la chambre de fermentation (10), de telle manière que les matières à fermenter (41) et la vapeur (43) se mélangent à proximité spatiale du dispositif d'alimentation (3).

2. Fermenteur (2) selon la revendication 1, **caractérisé en ce que** le dispositif d'alimentation (3) et ledit au moins un injecteur de vapeur (7) sont disposés à proximité l'un de l'autre.

3. Fermenteur (2) selon une des revendications 1 à 2, **caractérisé en ce que** l'injecteur de vapeur (7) est réalisé en forme de lance à vapeur.

4. Fermenteur (2) selon l'une quelconque des revendications 1 à 3, **caractérisé en ce que** le dispositif d'alimentation (3) est réalisé en forme de transporteur à vis sans fin pour des matières à fermenter au moins largement solides (41).

5. Fermenteur (2) selon l'une quelconque des revendications 1 à 4, **caractérisé en ce que** le fermenteur (2) est réalisé en forme de fermenteur avec écoulement en régime bouchon.

6. Installation de production de biogaz comportant au moins un fermenteur (2) selon l'une quelconque des revendications précédentes et un dispositif de fourniture de vapeur (5), qui est couplé à l'injecteur de vapeur (7) par une arrivée de vapeur (6).

7. Installation de production de biogaz selon la revendication 6, **caractérisée en ce qu'**il est prévu un générateur de vapeur fonctionnant au gaz pour la fourniture de vapeur.

8. Installation de production de biogaz selon la revendication 6 ou 7, **caractérisée en ce que** l'installation (1) présente une centrale de cogénération (24) avec un moteur à combustion interne, dans laquelle la centrale de cogénération (24) est couplée au dispositif de fourniture de vapeur (5), de telle manière que la chaleur perdue du moteur à combustion interne soit utilisée pour la production de vapeur.

9. Procédé de production de biogaz (55) par fermentation anaérobie de matières à fermenter organiques (41) dans un fermenteur (2) selon l'une quelconque des revendications 1 à 5, dans lequel on charge les matières à fermenter (41) au moyen d'un dispositif d'alimentation (3) dans une chambre de fermentation (10) du fermenteur (2),
**caractérisé en ce que** l'on fournit aux matières à fermenter (41) à proximité spatiale du dispositif d'alimentation (3) de l'énergie thermique par introduction de vapeur (43).

10. Procédé selon la revendication 9, **caractérisé en ce que** l'on effectue la fourniture d'énergie thermique lors de l'entrée des matières à fermenter (41) dans la chambre de fermentation (10).
